# EUROPÄISCHE PATENTSCHRIFT

(11) **EP 4 482 554 B1**
(45) Veröffentlichungstag und Bekanntmachung des Hinweises auf die Patenterteilung: **29.04.2026**
(21) Anmeldenummer: 23707368.9
(22) Anmeldetag: 24.02.2023
(51) Int. Cl.: A61M 11/00, A61M 13/00

(54) **MEDIZINISCHES INSTRUMENT ZUM EINBRINGEN EINER FLUIDFÖRMIGEN THERAPEUTISCHEN SUBSTANZ IN EINEN HOHLRAUM EINES KÖRPERS**
MEDICAL INSTRUMENT FOR INTRODUCING A FLUID THERAPEUTIC SUBSTANCE INTO A CAVITY OF A BODY
INSTRUMENT MÉDICAL POUR INTRODUIRE UNE SUBSTANCE THÉRAPEUTIQUE FLUIDE DANS UNE CAVITÉ D'UN CORPS

(30) Priorität: 25.02.2022 DE 102022104560
(43) Veröffentlichungstag der Anmeldung: 01.01.2025
(73) Patentinhaber: Capnopharm GmbH, 72070 Tübingen (DE)
(72) Erfinder: SOLASS, Wiebke, 72076 Tübingen (DE)
(74) Vertreter: Eisenführ Speiser
(86) Internationale Anmeldenummer: PCT/EP2023/054687
(87) Internationale Veröffentlichungsnummer: WO 2023/161419

(56) Entgegenhaltungen:
- EP-A2- 1 433 493
- WO-A1-2020/048627
- WO-A1-92/19383
- DE-A1- 102007 014 100
- DE-A1- 102012 104 629
- DE-A1- 102016 202 316
- JP-A- 2001 104 489

## Beschreibung

### Technisches Gebiet

Die Erfindung betrifft ein medizinisches Instrument zum Einbringen einer fluidförmigen therapeutischen Substanz in einen Hohlraum eines Körpers. Dabei weist das medizinische Instrument eine Düse mit einem Düsenkopf zum Zerstäuben der einzubringenden fluidförmigen therapeutischen Substanz auf. Ferner weist das medizinische Instrument einen zumindest im Wesentlichen länglichen und zumindest im Wesentlichen starren Schaft auf, an dessen einen Ende der Düsenkopf angeordnet ist. Weiterhin ferner weist das medizinische Instrument einen flexiblen Schlauch auf, der innerhalb des länglichen Schafts angeordnet ist und mit dem Düsenkopf fluidisch verbunden ist.

### Stand der Technik

Medizinische Instrumente zum Einbringen einer fluidförmigen therapeutischen Substanz in einen Hohlraum eines Körpers sind im Stand der Technik bekannt. Beispielsweise sind aus der DE 10 2012 104 629 A1 oder aus der WO 2020/048627 A1 solche Instrumente bekannt, die einen Schaft aufweisen, an dessen einem Ende eine Düse angeordnet ist, mit der die fluidförmige therapeutische Substanz in einen Hohlraum eines Körpers versprüht werden kann.

Die EP 1 433 493 A2 betrifft Vernebelungskathether, insbesondere zum Einbringen eines mit einem Gas zu einem Aerosol vernebelten Medikaments in eine Lunge. Aus der WO 92/19383 A1 ist es bekannt einen Prallkörper zum Zerstäuben bei Inhaliergeräten zu verwenden, und so eine feinere Tröpfchengröße zu erreichen. Die DE 10 2016 202316 A1 lehrt, submikrone Partikel mit einem Aerosolgenerator zu erzeugen und diese in einem Trägergasstrom in eine Körperhöhle einzubringen. Zerstäuben mittels eines Aufprallstifts ist aus der DE 10 2007 014100 A1 für andere Industrien, etwa zur Meerwasserentsalzung oder Goldgewinnung bekannt, wobei sich ein Verfahrensschritt zum Trennen von Komponenten eines Gasgemischs anschließt. Aus JP 2001 104489 A ist ein Vernebler mit einem Flüssigkeitsreflektor für ein Endoskop bekannt, das zum gleichmäßigen Verteilen einer Flüssigkeit an einer Darminnenwand ausgebildet ist.

Allerdings ist der Aufbau der bekannten Instrumente relativ komplex. Zudem ist die fluidförmige therapeutische Substanz mit den bekannten Instrumenten nicht optimal in dem Hohlraum des Körpers verteilbar. Ausserdem verlangen diese Technologien meistens einen Gasfluss, was die Anwendung in einer geschlossenen Körperhöhle erschwert: um einen Druckanstieg in der Körperhöhle zu vermeiden, muss ein Eingang und ein Ausgang geschaffen werden, wodurch der Anteil einer therapeutischen Lösung, der in der Körperhöhle verbleibt oder diese Körperhöhle nur durchläuft kaum ermittelt werden kann. Ausserdem ist es nicht möglich, die Geschlossenheit des Vernebelungssystems zu prüfen. So kann bei kontinuierlichem Gasfluss der Austritt einer toxischen Substanz (wie z.B. zytotoxischer Medikamenten) nicht ausgeschlossen werden. Ein weiteres Problem im Stand der Technik sind zum Teil hohe tote Volumina in den Verabreichungssystemen, die dazu führen können, dass 5 % bis 10 % der therapeutischen Substanz den Patienten nicht erreichen. Auch hier besteht ein Verbesserungsbedarf.

### Darstellung der Erfindung

Der Erfindung liegt die Aufgabe zugrunde, ein medizinisches Instrument zum Einbringen einer fluidförmigen therapeutischen Substanz in einen Hohlraum eines Körpers anzugeben, das die oben genannten Probleme und Nachteile des Standes der Technik ausräumt. Insbesondere ist es Aufgabe der vorliegenden Erfindung, ein medizinisches Instrument anzugeben, das einen besonders einfachen Aufbau hat und eine besonders gute Verteilung der therapeutischen Substanz in dem Hohlraum des Körpers bietet.

Diese Aufgabe wird durch den Gegenstand des unabhängigen Anspruchs 1 gelöst. Weitere mögliche Ausgestaltungen der Erfindung sind insbesondere in den abhängigen Ansprüchen angegeben.

Die erfindungsgemäße Lösung besteht insbesondere darin, ein medizinisches Instrument zum Einbringen einer fluidförmigen therapeutischen Substanz in einen Hohlraum eines Körpers anzugeben, das Folgendes aufweist: eine Düse mit einem Düsenkopf zum Zerstäuben der einzubringenden fluidförmigen therapeutischen Substanz ohne Zugabe von Gas; einen zumindest im Wesentlichen länglichen und zumindest im Wesentlichen starren Schaft, an dessen einem Ende der Düsenkopf angeordnet ist; und einen flexiblen Schlauch, der innerhalb des länglichen Schafts angeordnet ist und mit dem Düsenkopf fluidisch verbunden ist, wobei die Düse eine Aufpralleinrichtung aufweist, die einen Aufprallkörper aufweist, der vor einer Düsenöffnung des Düsenkopfes zumindest im Wesentlichen koaxial zu der Düsenöffnung angeordnet ist, wobei die Düsenöffnung einen Durchmesser in einem Bereich von 0,01 mm bis 0,5 mm aufweist, und wobei zumindest ein Teil der Aufpralleinrichtung einstückig mit dem Düsenkopf ausgebildet ist.

Als Hohlraum eines Körpers wird sowohl jeder körpereigene Hohlraum, wie beispielsweise ein natürlicher Hohlraum eines Hohlorgans oder eine Körperhöhle, beispielsweise Bauchhöhle oder Pleurahöhle, als auch jeder chirurgisch erzeugte Hohlraum, wie beispielweise ein Pseudopneumoperitoneum, verstanden. Häufig können Hohlorgane wie die Speiseröhre, der Magen, die Harnblase oder andere Organe, die z. B. einen virtuellen Raum enthalten und mit einem Epithel ausgekleidet sind, als Krebsherd dienen. Besonders im Hinblick auf Krebsherde sind die Bauchhöhle und die Pleurahöhlen wichtige Regionen des Körpers. Der Bauchraum erstreckt sich vom Thorax am Thoraxzwerchfell bis zum Becken am Beckenrand. Der Bauchraum wird als Bauchhöhle bezeichnet und beherbergt zahlreiche Organe wie den Magen, den Dünn- und Dickdarm, die Leber, die Gallenblase und die Bauchspeicheldrüse. Die Bauchhöhle und die Pleurahöhlen sind von einer ausgedehnten Membran ausgekleidet, die als Peritoneum bzw. Pleura bezeichnet wird und die Bauchwand und die Beckenwände (parietales Peritoneum) sowie die darin enthaltenen Organe (viszerales Peritoneum) bzw. die Innenfläche der Brusthöhle (parietales Pleura) sowie die darin enthaltenen Organe (viszerales Pleura) bedeckt. In diesen Bereichen kann sich beispielsweise ein Krebsort ausbreiten, d.h. dort kann sich das jeweilige Gewebe oder Teil des Gewebes befinden, in dem sich Krebszellen befinden.

Bei der fluidförmigen therapeutischen Substanz handelt es sich um ein Fluid, insbesondere ein Pharmakum, das im Rahmen einer therapeutischen Maßnahme bzw. Behandlung eines Patienten in den Hohlraum des Körpers des zu behandelten Patienten eingebracht wird. Das Fluid, vorzugsweise Pharmakum, ist insbesondere flüssig und nicht gasförmig. Erfindungsgemäß wird allein die fluidförmige (flüssige) therapeutische Substanz zerstäubt, ohne Zugabe von Gas. D.h., aus der Düse tritt lediglich eine zerstäube Flüssigkeit und kein Gas aus. Die fluidförmige therapeutische Substanz dient vorzugsweisedazu, den Krebsort zu behandeln. Vorzugsweise ist oder umfasst die fluidförmige therapeutische Substanz ein oder mehrere Chemotherapeutika. Vorzugsweise ist/sind das/die eine(n), zwei(n), mehrere(n) oder alle Chemotherapeutika, die im Rahmen der vorliegenden Erfindung verwendet werden, aus der Gruppe ausgewählt, die aus Chemotherapeutika besteht, die gegen einen Krebstyp verwendet werden, der aus der Gruppe ausgewählt ist, die umfasst: Magen-Darm-Krebs, insbesondere Magenkrebs, Dickdarmkrebs, Leber- oder Bauchspeicheldrüsenkrebs, Blinddarmkrebs, Speiseröhrenkrebs, Leberzellkarzinom, gynäkologischem Krebs; insbesondere primärer Peritonealkrebs, Eierstockkrebs, Endometriumkrebs; Prostatakrebs, Leukämie, Lymphom, Weichteilsarkom, Multiples Myelom, Blasenkrebs, Lungenkrebs, Schilddrüsenkrebs, Kaposi-Sarkom und Tumore embryonalen Ursprungs.

Das Volumen des bzw. jedes Wirkstoffs, vorzugsweise Chemotherapeutikums, aber auch DNA, RNA, Nanopartikeln, Immunotherapeutika und Viren, ist vorzugsweise in einem Volumen von 5 ml bis 2500 ml, vorzugsweise 10 ml bis 1000 ml, weiter bevorzugt 20 ml bis 500 ml, weiter bevorzugt 30 bis 300 ml fluidförmiger therapeutischer Substanz enthalten, wobei sich diese Angabe vorzugsweise auf die während einer Anwendung verabreichte Gesamtmenge bezieht, vorzugsweise gelöst oder suspendiert oder redispergiert in hydrophilen oder lipophilen Lösungen und Emulsionen.

Vorzugsweise ist eine Behandlung auf eine Krebsart gerichtet, die aus der Gruppe ausgewählt ist, die umfasst: Magen-Darm-Krebs, insbesondere Magenkrebs, kolorektalem Krebs, hepatobiliärem oder pankreatischem Krebs, Blinddarmkrebs, Speiseröhrenkrebs, hepatozellulärem Karzinom, oder gynäkologischer Krebs; insbesondere primärer Peritonealkrebs, Eierstockkrebs, Endometriumkrebs, und Tumore embryonalen Ursprungs. Die hier beschriebene Behandlung kann auf die Verringerung der Tumorgröße und der Anzahl der Tumorherde abzielen. Zu den Tumorherden gehören sowohl der Primärtumor als auch Metastasen und weitere Tumorvermehrungen. Die Behandlung kann auch darauf abzielen, das Wiederauftreten von Krebs nach einer Tumorentfernung oder Teilentfernung des Tumors, vorzugsweise nach einer chirurgischen Tumorentfernung, zu verhindern oder zu verzögern.

Es ist bevorzugt, dass die Behandlung wiederholt wird, mit zwei, drei oder mehr Verabreichungen der mit dem Verabreichungssystem aerosolisierten therapeutischen Substanz.

Im medizinischen Instrument bzw. Verabreichungssystem ist das Ende des Schlauchs insbesondere unmittelbar mit der Düse verbunden. Das andere Ende des Schlauchs ist mit einer Fluidquelle zum Bereitstellen der fluidförmigen therapeutischen Substanz fluidisch verbindbar oder verbunden. Der Schlauch erstreckt sich durch den Schaft hindurch bis zu dem Ende des Schafts, an dem die Düse angeordnet ist. So kann das «tote Volumen» der therapeutischen Substanz im Verabreichungssystem auf null reduziert werden. Vorzugsweise ist der Schlauch unmittelbar mit der Fluidquelle verbunden. Der Schlauch kann aber auch nur indirekt mit der Düse verbunden sein, insbesondere unter Zwischenschaltung eines Verbindungselements. Beispielsweise ist es auch denkbar, dass der Schaft selbst einen Hohlraum bildet, der einerseits mit einem Auslass des Schlauchs und andererseits mit einem Einlass der Düse verbunden ist, sodass der Schaft selbst Teil der fluidgängigen Verbindung ist. In diesem Fall kann aber das «tote Volumen» der therapeutischen Substanz, das den Patienten nicht erreicht, erhöht sein.

Im Verabreichungssystem kann eine Aufpralleinrichtung vorzugsweise an dem Düsenkopf angeordnet sein. Zumindest ein Teil der Aufpralleinrichtung ist einstückig mit dem Düsenkopf ausgebildet. Dass der Aufprallkörper vor der Düsenöffnung des Düsenkopfes angeordnet ist, bedeutet, dass der Aufprallkörper in einer Richtung weg von dem Schaft angeordnet ist. Der Aufprallkörper ist also so angeordnet, dass die Substanz, die aus der Düsenöffnung strömt, auf den Aufprallkörper trifft und mittels des Aufprallkörpers besser verteilt wird.

Einerseits sorgt der Aufprallkörper für einen besonders breiten Vernebelungswinkel. So kann beispielsweise ein Vernebelungswinkel, der größer ist als 120°, insbesondere größer ist als 150° erreicht werden.

Andererseits verändert der Aufprallkörper die besonders geringe Tröpfchengröße nicht So kann beispielsweise eine mediane aerodynamische Tröpfchengröße (MAD - median aerodynamic diameter) von kleiner als 35µm, insbesondere kleiner als 30µm erreicht werden. Diese Tröpfchengröße verändert sich kaum zwischen Wasser (aqua distillata), zuckerhaltigen Lösungen (wie z.B. Glc 5%) und öligen Lösungen (wie z.B. Silikonöl 5). Die Viskosität der fluidförmigen therapeutischen Substanz liegt vorzugsweise in einem Bereich von 1 bis 100 mPas, weiter bevorzugt 1 bis 30 mPas.

Insgesamt ergibt sich dank der Aufpralleinrichtung eine besonders gute Verteilung der therapeutischen Substanz in dem Hohlraum, bei einem gleichzeitigen einfachen Aufbau des medizinischen Instruments und ohne Beeinträchtigung der Funktion der Kegeldüse.

Gemäß einer vorteilhaften Weiterbildung der Erfindung weist der Aufprallkörper im Längsschnitt eine Parabelform, eine Dreieckform, eine Trapezform oder eine gestufte Pyramidenform auf. Der Längsschnitt ist ein Schnitt entlang einer Schnittebene, in der eine Rotationsachse des Instruments, die sich durch die Düsenöffnung erstreckt, liegt. Die Parabelform und die gestufte Pyramidenform sind besonders geeignet, um die Substanz gut zu verteilen. Vorteilhaft ist der Aufprallkörper rotationssymmetrisch, vorzugsweise um eine Achse, die koaxial mit der Düsenöffnung ist. Eine Parabelform ergibt hier dann einen ellipsoiden Körper, eine Dreieckform einen Kegel, eine gestufte Pyramidenform, einen gestuften Kegel, wobei die Stufen zylindrisch oder ebenfalls kegelig bzw. kegelstumpfförmig sein können.

Eine vorteilhafte Weiterbildung der Erfindung sieht vor, dass die Aufpralleinrichtung eine Halteeinrichtung aufweist, an der der Aufprallkörper angeordnet ist. Mittels der Halteeinrichtung kann somit der Aufprallkörper einfach an der richtigen Position an der Düse angeordnet werden. Die Halteeinrichtung ist vorzugsweise zumindest im Wesentlichen stabförmig dünn ausgebildet und weiterhin vorzugsweise um 90° oder 180° gebogen. Durch die stabförmige Ausbildung behindert die Halteeinrichtung das Verteilen der Substanz nur wenig. Die Halteeinrichtung kann auch mehr als einen Arm umfassen, sodass die einzelnen Arme dünner ausfallen können. Bei einer weiteren vorteilhaften Weiterbildung der Erfindung ist die Halteeinrichtung L-förmig oder U-förmig gebogen ausgebildet, wobei an einem ersten Ende der Halteeinrichtung der Aufprallkörper angeordnet ist, und wobei das zweite Ende der Halteeinrichtung radial außenliegend an dem Düsenkopf und/oder an dem Schaft angebracht ist.

Insbesondere ist die Halteinrichtung als L-förmig gebogener Stab ausgebildet, dessen Durchmesser vorzugsweise maximal 1/5 des Durchmessers des Düsenkopfes beträgt. Hierdurch blockiert die Halteeinrichtung das Verteilen der Substanz besonders wenig. Wenn mehrere Stäbe vorgesehen sind ist die Summe der Durchmesser der zwei oder mehr Stäbe vorzugsweise maximal 1/5 des Durchmessers des Düsenkopfes, vorzugsweise maximal 1/7, weiter bevorzugt maximal 1/10.

Unabhängig hiervon kann die Halteeinrichtung zu der Düsenöffnung hin spitzförmig zulaufend ausgebildet sein. Insbesondere kann die Halteeinrichtung hierbei spitzförmig zulaufende Ablenkflächen aufweisen, wobei die Ablenkflächen der Halteeinrichtung die Substanz ablenken, so dass es zu weniger Anhaftungen der Substanz an der Halteeinrichtung kommt.

Eine vorteilhafte Weiterbildung der Erfindung sieht vor, dass an der Halteeinrichtung eine Aufladeeinrichtung zum elektrostatischen Aufladen der fluidförmigen therapeutischen Substanz angeordnet ist.

Durch das elektrostatische Aufladen der fluidförmigen therapeutischen Substanz kann die Haftung und/oder die Eindringtiefe von Partikeln der Substanz an oder in Gewebe in dem Hohlraum verbessert werden.

Bei einer vorteilhaften Weiterbildung der Erfindung ist die Aufladeeinrichtung auf einer entgegengesetzten Seite zu dem Aufprallkörper an der Halteeinrichtung angeordnet.

Insbesondere ist ein Aufladebereich der Aufladeeinrichtung, der zur elektrostatischen Aufladung ausgebildet ist, auf der entgegengesetzten Seite zu dem Aufprallkörper an der Halteeinrichtung angeordnet. Somit liegt die Aufladeeinrichtung bzw. der Aufladebereich hiervon nicht im direkten Sprühfeld der Düse. Hierdurch wird verhindert, dass die Aufladeeinrichtung bzw. der Aufladebereich hiervon durch die Substanz bzw. Ablagerungen hiervon bedeckt wird. Die Aufladeeinrichtung bzw. der Aufladebereich kann allgemein bürstenartig oder aus Vollmaterial ausgebildet sein. Bei einer bürstenartigen Ausbildung ist der Aufladebereich mit mehreren elektrischen Drähten ausgebildet. Bei einer Ausbildung als Vollmaterial ist der Aufladebereich einförmig ausgebildet. Insbesondere bei einer einförmigen Ausbildung kann der Aufladebereich besonders bevorzugt bündig mit der Halteeinrichtung ausgebildet sein.

Gemäß einer vorteilhaften Weiterbildung der Erfindung weist die Aufladeeinrichtung eine Leiterfolie auf oder ist aus einer Leiterfolie ausgebildet. Eine solche Leiterfolie kann als Ausbildung der Aufladeeinrichtung bzw. des Aufladebereichs aus Vollmaterial verstanden werden. Die Leiterfolie erstreckt sich hierbei vorzugsweise von dem einen Ende des Schafts, an dem die Düse angeordnet ist, entlang des Schafts hin zu dem anderen Ende des Schafts, das von der Düse entfernt angeordnet ist. An dem anderen Ende ist die Leiterfolie mittels eines Anschlusses mit einer Energiequelle, insbesondere mit einer Hochspannungsquelle, verbindbar oder verbunden. An dem einen Ende des Schafts erstreckt sich die Leiterfolie von dem Schaft entlang der Düse bzw. entlang des Düsenkopfs und der Halteeinrichtung der Aufpralleinrichtung.

Durch die Leiterfolie ist es einfach und sicher möglich, die Aufladung der Substanz an dem Aufladebereich der Aufladeeinrichtung bereitzustellen. Außerdem ist die Leiterfolie äußerst flach, so dass die maximale Baugröße des Instruments, beispielsweise zur Verwendung in einem Trokar, nicht überschritten wird.

Eine vorteilhafte Weiterbildung der Erfindung sieht vor, dass ein Bereich der Leiterfolie zum Ausbilden eines Aufladebereichs zum elektrostatischen Aufladen der fluidförmigen therapeutischen Substanz freiliegt.

Mit anderen Worten liegt an dem Aufladebereich ein elektrischer Leiter der Leiterfolie derart frei, dass dieser zum elektrostatischen Aufladen der fluidförmigen therapeutischen Substanz ausgebildet ist. Der freiliegende Bereich ist also dazu ausgebildet, eine elektrostatische Ladung an die therapeutische Substanz bzw. Partikel hiervon, insbesondere während einer Behandlung, abzugeben. Beispielsweise kann eine Isolationsfolie der Leiterfolie an dem Aufladebereich entfernt werden, so dass der elektrische Leiter zum Kontaktieren der Substanz freiliegt.

Bei einer vorteilhaften Weiterbildung der Erfindung weist der Düsenkopf einen Steckanschluss an einem der Düsenöffnung entgegengesetzten Ende auf, wobei der Schlauch direkt auf den Steckanschluss aufgesteckt ist. Da der Schlauch direkt mit dem Düsenkopf bzw. mit dessen Steckanschluss verbunden ist, wird ein einfach ausgebildetes Instrument erreicht, bei dem zum Hinleiten der therapeutischen Substanz zum Düsenkopf nur eine einzige Verbindungsstelle nötig ist. So kann die Anzahl an Verbindungsstellen und damit die hieraus resultierenden Schwachstellen, wie Undichtigkeit oder Druckverlust, gering gehalten werden. Ferner hat die Substanz so nur Kontakt mit der Düse und dem Schlauch, so dass Verunreinigungen vermindert werden können. Außerdem ist der Steckanschluss ohne aufwendige Dichtmittel abdichtbar. Ein einfaches Abdichten ergibt sich insbesondere, wenn der Schlauch mit einer Presspassung auf den Steckanschluss aufgeschoben ist. Der Schlauch ist daher vorzugsweise derart flexibel, dass dieser sich beim Aufschieben auf den Steckanschluss radial aufweitet. Um das Aufschieben zu erleichtern, kann der Steckanschluss vorzugsweise an seinem dem Schlauch zugewandten Außenumfang eine Fase aufweisen. Zudem ist es leichter möglich, verschiedene Varianten des medizinischen Instruments angepasst für spezifische Substanzen bereitzustellen.

Der Düsenkopf kann mit dem Steckanschluss einstückig ausgebildet sein. Dann weist dieser auch eine durchgehende vorzugsweise gerade Bohrung zur Leitung der fluidförmigen therapeutischen Substanz auf. Alternativ hierzu wäre es allerdings denkbar, dass der Düsenkopf mehrstückig, beispielsweise zweistückig, ausgebildet ist. Dann können einfach weitere Funktionselemente in die Düse integriert werden. Bei einer zweistückigen Variante, bei der der Steckanschluss und der Rest des Düsenkopfs in zwei Einzelteilen vorliegen, kann der Steckanschluss vorzugsweise in den Düsenkopf gepresst werden. Besonders vorzugsweise weist der Steckanschluss und/oder der Düsenkopf hierbei mehrere radial umlaufende Dichtrippen auf. Hierdurch kann auf eine weitere Abdichtung verzichtet werden.

Gemäß einer vorteilhaften Weiterbildung der Erfindung weist das medizinische Instrument eine Klemmhülse auf, die derart an dem Schlauch angeordnet ist, dass die Klemmhülse das auf dem Steckanschluss aufgesteckte Ende des Schlauchs umlaufend mit ihrer inneren Mantelfläche gegen die äußere Mantelfläche des Steckanschlusses presst. Hierdurch kann ein abdichtendes Halten des Schlauchs auf dem Steckanschluss erreicht werden. Die Klemmhülse erzeugt von außen einen Gegendruck auf das aufgesteckte Ende des Schlauchs, so dass das Ende des Schlauchs dicht auf dem Steckanschluss sitzt.

Eine vorteilhafte Weiterbildung der Erfindung sieht vor, dass der Schaft einen vorzugsweise umlaufenen Anschlagsbereich aufweist, an dem die Klemmhülse anschlägt. Durch den Anschlagbereich wird eine genaue Vorgabe der Position, insbesondere der axialen Position - gesehen in Richtung der Rotationsachse -, der Klemmhülse ermöglicht. Gleichzeitig sorgt der Anschlagbereich dafür, dass ein insbesondere axiales Verschieben der Klemmhülse verhindert wird.

Vorzugsweise weist auch die Düse einen entsprechenden weiteren Anschlagbereich auf. Dann ist die Klemmhülse in dem montierten Zustand des Instruments insbesondere axial zwischen dem Anschlagbereich des Schafts und dem weiteren Anschlagbereich der Düse angeordnet.

In anderen Ausführungsformen ist der Schlauch in den Düsenkopf eingesteckt. Hierzu kann der Düsenkopf eine Schlauchaufnahmeöffnung aufweisen, in die der Schlauch eingesteckt werden kann. Beispielsweise kann der Schlauch auch durch geeignete Schweißverfahren mit dem Düsenkopf verbunden werden. Verschraubungen und Verklebungen können vermieden werden, wodurch die Sicherheit verbessert und auch die Herstellung vereinfacht werden kann.

Bei einer weiteren vorteilhaften Weiterbildung der Erfindung weist das medizinische Instrument einen Filter bzw. Partikelfilter auf, der vorzugsweise in der Düse angeordnet ist. Bei dem Filter bzw. Partikelfilter kann es sich um ein vorab als Funktionselement bezeichnetes Bauteil handeln. Entsprechend kann dann die Düse insbesondere eine zweistückige Ausbildung aufweisen. Bei dem Partikelfilter kann es sich beispielsweise um einen Polypropylen- oder Metall-Filter handeln. Der Filter ist insbesondere zum Reinigen der fluidförmigen therapeutischen Substanz von Unreinheiten angeordnet. Alternativ hierzu wäre es auch denkbar, dass der Filter in dem Schlauch, vorzugsweise an dem dem Düsenkopf zugewandten Ende des Schlauchs angeordnet ist. Er kann aber auch eingangsseitig am Schlauch angeordnet oder integraler Teil des Schlauchs sein.

Gemäß einer vorteilhaften Weiterbildung der Erfindung ist eine Bohrung in dem Schaft zu einem Außendurchmesser des Schlauchs als Spielpassung derart ausgebildet, dass der Schlauch innerhalb des Schafts bewegbar, insbesondere drehbar, angeordnet ist. Hierdurch wird die Handhabung des medizinischen Instruments verbessert, da der Schlauch sich hierbei mitdrehen kann.

Bei einer weiteren vorteilhaften Weiterbildung der Erfindung weist das medizinische Instrument ferner einen verstellbaren Außenanschlag zur Positionierung in einem Trokar und einen Griff auf, die an dem Schaft angeordnet sind. Durch den Außenanschlag kann die korrekte Positionierung innerhalb des Trokars einfach sichergestellt werden. Der Griff verbessert die Handhabung des medizinischen Instruments. An dem Außenanschlag kann vorzugsweise ein Abführungsbereich integriert sein, der beispielsweise einen Abfuhranschluss zum Verbinden mit einer Leitung aufweist. Der Abführungsbereich dient beispielsweise dazu, nach der Operation ein toxisches Aerosol sicher abzuführen.

Gemäß einer vorteilhaften Weiterbildung der Erfindung weist die Düse eine maschinenlesbare Kennung auf, in der Informationen zur Art der Düse enthalten sind. Bei den Informationen kann es sich beispielsweise um Informationen zu den mit der Düse verwendbaren Substanzen handeln. So können unterschiedliche Düsen, die sich von anderen Düsen beispielsweise in der Größe der Düsenöffnung oder der Ausbildung der Aufpralleinrichtung unterscheiden, für die Verwendung mit unterschiedlichen Substanzen geeignet sein. Bei der maschinenlesbaren Kennung kann es sich insbesondere um einen Chip oder um einen Barcode handeln.

Beispielsweise kann der Düsenkopf auswechselbar an dem Schaft angeordnet sein und insbesondere mittels eines Gewindes und/oder einer Rasteinrichtung an dem Schaft, vorzugsweise mittels eines Spezialwerkzeugs, lösbar befestigt sein. Hierdurch können mit einem Instrument mehrere Düsen verwendet werden. Somit ergibt sich hieraus auch ein System zum Einbringen einer therapeutischen Substanz in einen Hohlraum eines Körpers, wobei das System Folgendes aufweist: eines der vorab beschriebenen medizinischen Instrumente; und eine weitere Düse mit einem weiteren Düsenkopf und einer weiteren Aufpralleinrichtung mit einem weiteren Aufprallkörper, wobei der weitere Düsenkopf eine weitere Düsenöffnung aufweist, wobei die weitere Düse anstelle der Düse an den Schaft anbringbar ist, wobei die Düsenöffnung des Düsenkopfs von der weiteren Düsenöffnung des weiteren Düsenkopfs und/oder der Aufprallkörper des Düsenkopfs von dem weiteren Aufprallkörper verschieden ist.

Auch wenn der Düsenkopf vorab als austauschbar und hierzu von dem Schaft zerstörungsfrei lösbar beschrieben worden ist, kann der Düsenkopf in einer weiteren bevorzugten Ausführungsform fest und insbesondere nicht zerstörungsfrei mit dem Schaft verbunden sein. Beispielsweise kann der Düsenkopf in den Schaft gepresst sein. Hierzu kann der Schaft und/oder der Düsenkopf vorzugsweise mehrere radial umlaufende Dichtrippen aufweisen, die beim Verpressen entsprechend verformt werden.

Die vorab beschriebenen erfindungsgemäßen medizinischen Instrumente stellen insgesamt ein einfach aufgebautes und gleichzeitig sicheres Werkzeug für die minimal-invasive Chirurgie und die Verabreichung von therapeutischen Aerosolen in Körperhöhlen bereit. Das erfindungsgemäße Instrument wird auch in einem System zum Einbringen von fluidförmigen therapeutischen Substanzen in Hohlräume bereitgestellt. Das System weist vorzugsweise einen Trokar, vorzugsweise eine Quelle für die therapeutische Substanz, vorzugsweise eine Fluidpumpe sowie eines der vorab beschriebenen erfindungsgemäßen Instrumente auf, das mit der Fluidquelle und der Fluidpumpe, insbesondere mittels des Schlauchs, fluidisch verbunden oder verbindbar ausgebildet ist und mittels des Trokars in den Hohlraum eingeführt werden kann.

In einem zweiten Aspekt löst die Erfindung die eingangs genannte Aufgabe durch ein System mit einem Trokar und einem medizinischen Instrument nach einer der vorstehend beschriebenen bevorzugten Ausführungsformen eines medizinischen Instruments gemäß dem ersten Aspekt, wobei sich das medizinische Instrument durch den Trokar erstreckt.

In einem dritten Aspekt (der nicht beansprucht wird) löst die vorliegende Offenbarung die Aufgabe bei einem therapeutischen Verfahren zum Behandeln von Tumorgewebe in einem Hohlorgan, durch die Schritte: Bereitstellen eines Trokars mit darin aufgenommenem medizinischen Instrument nach einer der vorstehenden beschriebenen bevorzugten Ausführungsformen eines medizinischen Instruments gemäß dem ersten Aspekt der Erfindung; Zuführen einer fluidförmigen therapeutischen Substanz zu dem medizinischen Instrument; und Zerstäuben der fluidförmigen therapeutischen Substanz mittels der Düse mit einem Vernebelungswinkel von größer 120°. Der Vernebelungswinkel ist vorzugsweise größer 150°, weiter bevorzugt größer 170°, 180°, 200°, 240°, 270°.

Vorzugsweise wird dem medizinischen Instrument ausschließlich die fluidförmige therapeutische Substanz zugeführt. Das heißt, dem medizinischen Instrument wird insbesondere kein Gas und/oder keine Treiber- und/oder Trägerflüssigkeit zugegeben.

Vorzugsweise wird die fluidförmige therapeutische Substanz mit einem Druck in einem Bereich von 10 bar bis 25 bar, vorzugsweise 11 bar bis 20 bar (gemessen vor der Düse) zugeführt. Hierdurch kann die Vernebelung der fluidförmigen Substanz vorteilhaft beeinflusst werden.

In einem weiteren Aspekt (der nicht beansprucht wird) löst die vorliegende Offenbarug die eingangs genannte Aufgabe durch eine Verwendung eines medizinischen Instruments nach einer der vorstehend beschriebenen bevorzugten Ausführungsformen eines medizinischen Instruments gemäß dem ersten Aspekt der Erfindung zum Behandeln von Tumorgewebe in einem Hohlorgan.

### Kurze Beschreibung der Zeichnungen

Die oben beschriebenen, unterschiedlichen und beispielhaften Merkmale können erfindungsgemäß miteinander kombiniert werden, soweit dies technisch sinnvoll und geeignet ist. Weitere Merkmale, Vorteile und Ausführungsformen der Erfindung ergeben sich aus der nachfolgenden Beschreibung von Ausführungsbeispielen und anhand der Figuren. Es zeigen:
Fig. 1 eine schematische perspektivische Darstellung eines exemplarischen medizinischen Instruments gemäß der vorliegenden Erfindung;
Fig. 2 eine Schnittansicht des in Fig. 1 gezeigten medizinischen Instruments;
Fig. 3 eine schematische vergrößerte Darstellung eines vorderen Bereichs des in Fig. 1 und Fig. 2 gezeigten medizinischen Instruments;
Fig. 4 eine Schnittansicht des in Fig. 3 gezeigten vergrößerten Bereichs des medizinischen Instruments; und
Fig. 5 eine schematische Anwendung eines medizinischen Instruments gemäß der vorliegenden Erfindung.

### Wege zur Ausführung der Erfindung

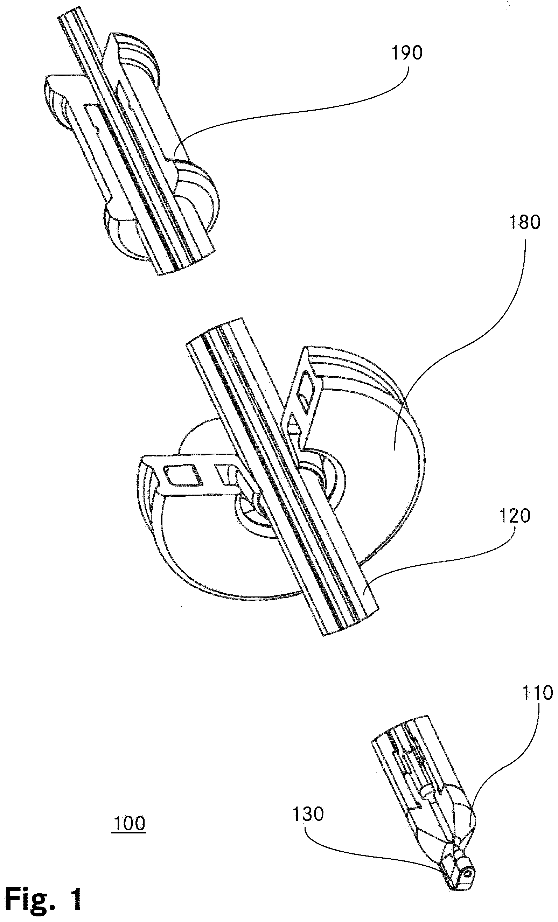

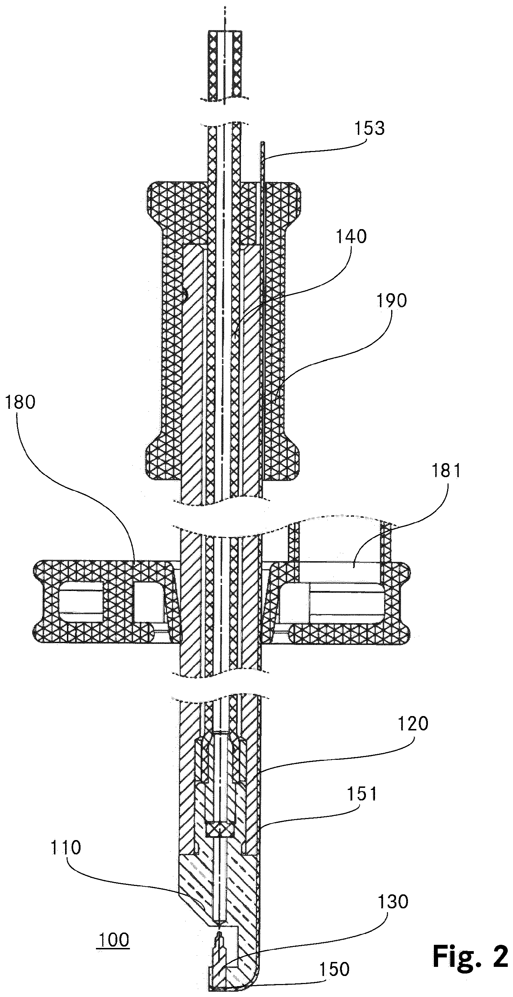

Fig. 1 zeigt eine schematische perspektivische Darstellung eines exemplarischen medizinischen Instruments 100 gemäß der vorliegenden Erfindung. In Fig. 2 ist eine Schnittansicht des in Fig. 1 gezeigten medizinischen Instruments 100 gezeigt.

Wie in der Fig. 1 und der Fig. 2 zu erkennen, weist das medizinische Instrument 100 einen länglichen Schaft 120 auf. An einem Ende des Schafts 120 ist eine Düse 110 mit einer Aufpralleinrichtung 130 ausgebildet. Für eine übersichtlichere Darstellung ist das medizinische Instrument 100 mit mehreren Brüchen dargestellt, um trotz der Länge des medizinischen Instruments 100 alle Aspekte auf einen Blick zu haben. An dem Schaft 120 sind ein Griff 190 und ein Außenanschlag 180 angeordnet. Der Außenanschlag 180 dient als Anschlag für einen nicht dargestellten Trokar 200.

In Fig. 2 ist zu erkennen, dass der Außenanschlag 180 eine weitere Funktion mittels eines Abführungsbereichs 181 ausführen kann. So kann der Außenanschlag 180 auch dazu dienen, mittels des Abführungsbereichs 181 ein Fluid, beispielsweise Insuffliergas, abzuführen.

Wie ebenfalls in Fig. 2 zu erkennen, verläuft innerhalb des Schafts 120 ein Schlauch 140 bis hin zu der Düse 110. Der Schlauch 140 erstreckt sich an dem zu der Düse 110 entgegengesetzten Ende aus dem Schaft 120 heraus und kann vorzugsweise bis zu einer nicht dargestellten Quelle 400 der therapeutischen Substanz geführt werden.

Die Aufladeeinrichtung 150 weist eine Leiterfolie 151 auf. Die Leiterfolie 151 erstreckt sich entlang des Schafts 120 bis hin zu dem zu der Düse 110 entgegengesetzten Ende. Insbesondere erstreckt sich die Leiterfolie 151 außen entlang des Schafts 120 bis zu dem entgegengesetzten Ende. An diesem entgegengesetzten Ende kann beispielsweise ein Anschluss 153 ausgebildet sein, um die Aufladeeinrichtung 150 mit einer nicht dargestellten Energiequelle 300 zum Bereitstellen der für die elektrostatische Aufladung benötigten Energie zu verbinden.

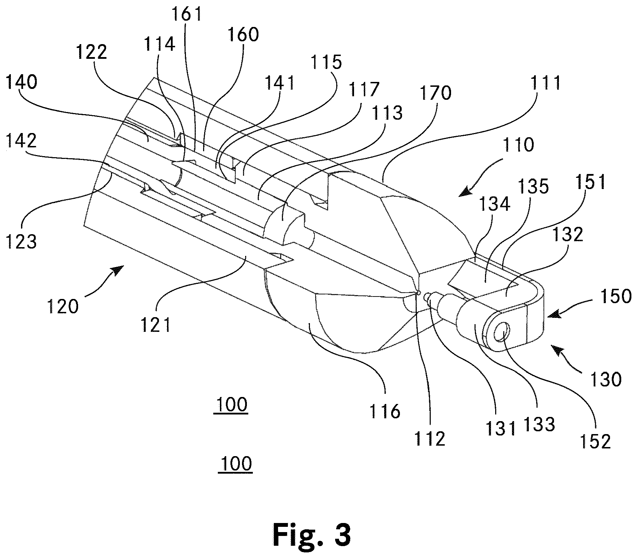

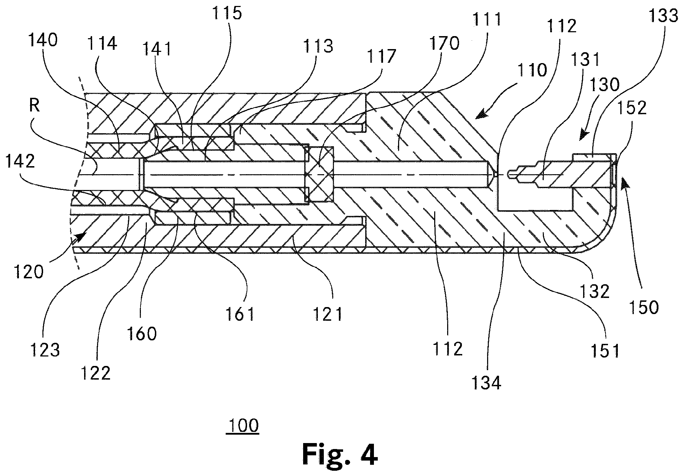

In der Fig. 3 ist eine schematische vergrößerte Darstellung eines vorderen Bereichs des in den Figs. 1 und 2 gezeigten medizinischen Instruments 100 gezeigt. Die Fig. 4 zeigt eine Schnittansicht des in Fig. 3 gezeigten vergrößerten Bereichs des medizinischen Instruments 100.

Die Düse 110 weist einen Düsenkopf 111 auf. Der Düsenkopf 111 ist an einem Ende 121 des Schafts 120 angeordnet. Hier ist der Düsenkopf 111 zweiteilig mit einem Steckanschluss 113 ausgebildet. Alternativ hierzu wäre auch ein mit einem Steckanschluss 113 einstückig ausgebildeter Düsenkopf 111 möglich. Der Steckanschluss 113 dient zur Verbindung mit dem Schlauch 140, insbesondere mit einem Ende 141 des Schlauchs 140. Hierzu wird das Ende 141 auf den Steckanschluss 113 aufgeschoben. Um das Aufschieben zu vereinfachen, weist der Steckanschluss 113 eine Fase 114 auf. Beim Aufschieben weitet sich der Schlauch 140 bzw. dessen Ende 141 radial auf.

Ferner weist der Düsenkopf 111 eine Düsenöffnung 112 auf. Die Düsenöffnung 112 ist an einem dem Steckanschluss 113 entgegengesetzten Ende des Düsenkopfs 111 angeordnet. Ebenfalls an diesem entgegengesetzten Ende ist die Aufpralleinrichtung 130 angeordnet. Die Aufpralleinrichtung 130 weist einen Aufprallkörper 131 auf, der dazu dient, die Substanz besser zu verteilen. Der Aufprallkörper 131 ist koaxial zu der Düsenöffnung 112 angeordnet, so dass die aus der Düsenöffnung 112 ausströmende fluidförmige Substanz auf den Aufprallkörper 131 auftrifft. Hier ist der Aufprallkörper 131 in einer stufenförmigen Pyramidenform ausgebildet. Alternativ hierzu könnte dieser beispielsweise auch parabelförmig ausgebildet sein.

Die Düsenöffnung hat einen Durchmesser in einem Bereich von 0,01 mm bis 0,5 mm, bevorzugt 0,02 mm bis 0,4 mm, weiter bevorzugt 0,05 mm bis 0,3 mm.

Die Aufpralleinrichtung 130 weist eine Halteeinrichtung 132 auf, um den Aufprallkörper 131 an der richtigen Position d.h. koaxial zu der Düsenöffnung 112 anzuordnen. Die Halteeinrichtung 132 weist ein erstes Ende 133 auf, an dem der Aufprallkörper 131 angeordnet ist, und ein zweites Ende 134 auf, an dem die Halteeinrichtung 132 mit dem Düsenkopf 111 verbunden ist.

Beispielsweise kann, wie in der Fig. 4 zu erkennen, die Halteeinrichtung 132 einstückig mit dem Düsenkopf 111 ausgebildet sein. Die Halteeinrichtung 132 erstreckt sich allgemein von dem Düsenkopf 111 aus zunächst in Axialrichtung nach vorne (in der Fig. 4 nach rechts) und dann zu einer Rotationsachse R des medizinischen Instruments 100, die auch durch die Düsenöffnung 112 hindurch verläuft, hin nach innen. Wie in der Fig. 3 zu erkennen, kann die Halteeinrichtung 132 hierbei eine oder mehrere Ablenkflächen 135 aufweisen, um die Substanz abzulenken und so ein Ablagern der Substanz an der Halteeinrichtung 132 zu reduzieren.

Insgesamt kann die Halteeinrichtung 132 wie in den Figs. 3 und 4 gezeigt zumindest im Wesentlichen L-förmig ausgebildet sein. Alternativ hierzu kann die Halteeinrichtung 132 auch U-förmig ausgebildet sein.

An der Düse 110 bzw. an dem Düsenkopf 111 ist weiterhin ein Teil der Aufladeeinrichtung 150 angeordnet. Insbesondere ist ein Aufladebereich 152 der Aufladeeinrichtung 150 an der Halteeinrichtung 132 angeordnet. Der Aufladebereich 152 ist beispielsweise, wie in der Fig. 3 angedeutet, ein freiliegender Bereich der Leiterfolie 151, die sich bis zu dem ersten Ende 133 der Halteeinrichtung 132 erstreckt. Der Aufladebereich 152 ist insbesondere auf einer dem Aufprallkörper 131 entgegensetzten Seite der Halteeinrichtung 132 angeordnet. Somit ist der Aufladebereich 152 auch auf einer der Düsenöffnung 112 abgewandten Seite der Halteeinrichtung 132 angeordnet. Dies hat den Vorteil, dass sich an dem Aufladebereich 152 weniger Partikel der fluidförmigen Substanz ablagern, die die Funktion der Aufladeeinrichtung 150 behindern könnten.

Innerhalb der Düse 110 ist ferner ein Partikelfilter 170 angeordnet. Dabei ist der Partikelfilter 170 insbesondere zwischen dem Steckanschluss 113 und dem Rest des Düsenkopfs 111 angeordnet.

Ferner weist das medizinische Instrument 100 eine Klemmhülse 160 auf, die dazu ausgebildet ist den Schlauch 140 bzw. dessen Ende 141 auf den Steckanschluss 113 zu pressen. Insbesondere presst hierbei eine innere Mantelfläche 161 der Klemmhülse 160 den Schlauch 140 auf eine äußere Mantelfläche 115 des Steckanschlusses 113. In axialer Richtung ist die Klemmhülse 160 zwischen einem Anschlagbereich 122 des Schafts 120 und einem weiterer Anschlagbereich 117 der Düse 110 angeordnet. Hierdurch kann ein Verrutschen der Klemmhülse 160 verhindert werden.

Wie in der Fig. 4 deutlich zu erkennen, ist zwischen dem Schlauch 140 und dem Schaft 120 ein Spiel ausgebildet, so dass der Schlauch 140 nicht innerhalb des Schafts 120 verklemmt. Hierzu ist eine Bohrung 123 des Schafts 120 größer als ein Außendurchmesser 142 des Schlauchs140 ausgebildet.

Das medizinische Instrument 100 kann eine Kennung 116 aufweisen. Bei der Kennung 116 kann es sich insbesondere um eine maschinenlesbare Kennung handeln, in der Informationen zur Art der Düse 110 enthalten sind.

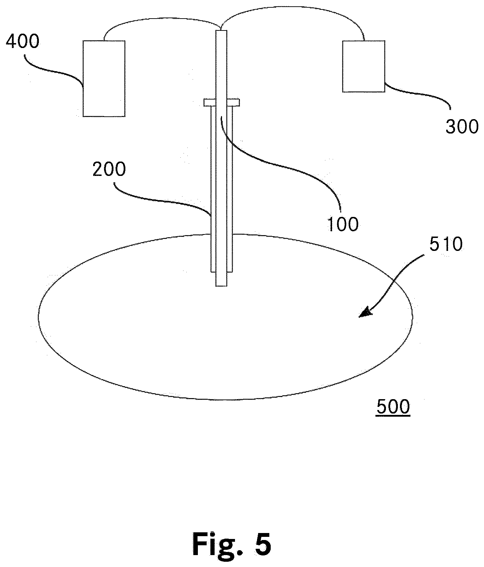

Fig. 5 zeigt die schematische Anwendung des medizinischen Instruments 100 gemäß der vorliegenden Erfindung. Das medizinische Instrument 100 ist zum Einbringen einer fluidförmigen therapeutischen Substanz in einen Hohlraum 510 eines Körpers 500 ausgebildet. Hierzu kann das medizinische Instrument 100 mittels eines Trokars 200 innerhalb des Hohlraums 510 angeordnet werden. Das medizinische Instrument100 ist, wie in der Fig. 5 angedeutet, mit einer Quelle 400, die die fluidförmige therapeutische Substanz bereitstellt, verbunden. Ferner ist das medizinische Instrument100 mit einer Energiequelle 300 zum Bereitstellen der Energie für die Aufladeeinrichtung 150 verbunden. Die Energiequelle ist vorzugsweise dazu ausgebildet eine Spannung in einem Bereich von 4 kV bis 9 kV, vorzugsweise 6 kV bis 7 kV bereitzustellen, und die Aufladeeinrichtung 150 ist dazu ausgebildet, eine derartige Spannung aufzunehmen.

Es ist festzuhalten, dass die unter Bezug auf einzelne Ausführungsformen bzw. -varianten beschriebenen Merkmale der Erfindung, wie beispielsweise Art und Ausgestaltung der einzelnen Komponenten sowie deren genaue Dimensionierung und räumliche Anordnung auch bei anderen Ausführungsformen vorhanden sein können. Von derartigen, in Kombination beschriebenen Merkmalen einzelner Ausführungsformen müssen außerdem nicht notwendigerweise alle Merkmale in einer betreffenden Ausführungsform realisiert sein.

## Patentansprüche

1. Medizinisches Instrument (100) zum Einbringen einer fluidförmigen therapeutischen Substanz in einen Hohlraum (510) eines Körpers (500), wobei das medizinische Instrument (100) folgendes aufweist:
eine Düse (110) mit einem Düsenkopf (111) zum Zerstäuben der einzubringenden fluidförmigen therapeutischen Substanz ohne Zugabe von Gas;
einen zumindest im Wesentlichen länglichen und zumindest im Wesentlichen starren Schaft (120), an dessen einem Ende (121) der Düsenkopf (111) angeordnet ist; und
einen flexiblen Schlauch (140), der innerhalb des länglichen Schafts (120) angeordnet ist und mit dem Düsenkopf (111) fluidisch verbunden ist,
**dadurch gekennzeichnet, dass**
die Düse (110) eine Aufpralleinrichtung (130) aufweist, die einen Aufprallkörper (131) aufweist, der vor einer Düsenöffnung (112) des Düsenkopfes (111) zumindest im Wesentlichen koaxial zu der Düsenöffnung (112) angeordnet ist,
wobei die Düsenöffnung (112) einen Durchmesser in einem Bereich von 0,01 mm bis 0,5 mm aufweist, und
wobei zumindest ein Teil der Aufpralleinrichtung (130) einstückig mit dem Düsenkopf (111) ausgebildet ist.

2. Medizinisches Instrument (100) nach Anspruch 1, wobei der Aufprallkörper (131) im Längsschnitt eine Parabelform, dreieckig, trapezförmig oder eine Pyramidenform, vorzugsweise gestufte Pyramidenform, aufweist.

3. Medizinisches Instrument (100) nach einem der Ansprüche 1 oder 2, wobei die Aufpralleinrichtung (130) eine Halteeinrichtung (132) aufweist, an der der Aufprallkörper (131) angeordnet ist, wobei die Halteeinrichtung (132) vorzugsweise L-förmig oder U-förmig gebogen ausgebildet ist, wobei an einem ersten Ende (133) der Halteeinrichtung (132) der Aufprallkörper (131) angeordnet ist, wobei das zweite Ende (134) der Halteeinrichtung (132) an dem Düsenkopf (111) und/oder an dem Schaft (120) angebracht ist.

4. Medizinisches Instrument (100) nach Anspruch 3, wobei an der Halteeinrichtung (132) eine Aufladeeinrichtung (150) zum elektrostatischen Aufladen der fluidförmigen therapeutischen Substanz angeordnet ist.

5. Medizinisches Instrument (100) nach Anspruch 4, wobei die Aufladeeinrichtung (150) auf einer entgegengesetzten Seite zu dem Aufprallkörper (131) an der Halteeinrichtung (132) angeordnet ist.

6. Medizinisches Instrument (100) nach Anspruch 4 oder 5, wobei die Aufladeeinrichtung (150) eine Leiterfolie (151) aufweist.

7. Medizinisches Instrument (100) nach Anspruch 6, wobei ein Bereich der Leiterfolie (151) zum Ausbilden eines Aufladebereichs (152) zum elektrostatischen Aufladen der fluidförmigen therapeutischen Substanz freiliegt.

8. Medizinisches Instrument (100) nach einem der vorherigen Ansprüche, wobei der Düsenkopf (111) einen Steckanschluss (113) an einem zu der Düsenöffnung (112) entgegengesetzten Ende aufweist, und wobei der Schlauch (140) direkt auf den Steckanschluss (113) aufgesteckt ist.

9. Medizinisches Instrument (100) nach Anspruch 8, wobei das medizinische Instrument (100) eine Klemmhülse (160) aufweist, die derart an dem Schlauch (140) angeordnet ist, dass die Klemmhülse (160) ein auf dem Steckanschluss (113) aufgestecktes Ende (141) des Schlauchs (140) umlaufend mit ihrer inneren Mantelfläche (161) gegen eine äußere Mantelfläche (115) des Steckanschlusses (113) presst.

10. Medizinisches Instrument (100) nach Anspruch 9, wobei der Schaft (120) einen vorzugsweise umlaufenen Anschlagsbereich (122) aufweist, an dem die Klemmhülse (160) anschlägt.

11. Medizinisches Instrument (100) nach einem der vorherigen Ansprüche, wobei das medizinische Instrument (100) einen Partikelfilter (170) aufweist, der vorzugsweise in der Düse (110) angeordnet ist.

12. Medizinisches Instrument (100) nach einem der vorherigen Ansprüche, wobei eine Bohrung (123) im Schaft (120) zu einem Außendurchmesser (142) des Schlauchs (140) als Spielpassung derart ausgebildet, dass der Schlauch (140) innerhalb des Schafts (120) bewegbar, insbesondere drehbar, angeordnet ist.

13. Medizinisches Instrument (100) nach einem der vorherigen Ansprüche, wobei das medizinische Instrument (100) ferner einen verstellbaren Außenanschlag (180) zur Positionierung in einem Trokar (200) und einen Griff (190) aufweist, die an dem Schaft (120) angeordnet sind.

14. Medizinisches Instrument (100) nach einem der vorherigen Ansprüche, wobei die Düse (110) eine maschinenlesbare Kennung (116) aufweist, in der Informationen zur Art der Düse (110) enthalten sind.

15. System mit einem Trokar (200) und einem medizinischen Instrument (100) nach Anspruch 13, wobei sich das medizinische Instrument (100) durch den Trokar (200) erstreckt.

## Claims

1. Medical instrument (100) for introducing a fluid therapeutic substance into a cavity (510) of a body (500), wherein the medical instrument (100) comprises:
a nozzle (110) with a nozzle head (111) for atomizing the fluid therapeutic substance to be introduced without adding gas;
an at least substantially elongated and at least substantially rigid shaft (120), at one end (121) of which the nozzle head (111) is arranged; and
a flexible tube (140) arranged inside the elongated shaft (120) and fluidically connected to the nozzle head (111),
**characterized in that**
the nozzle (110) has an impact device (130) comprising an impact body (131) arranged in front of a nozzle opening (112) of the nozzle head (111) at least substantially coaxially with the nozzle opening (112),
wherein the nozzle opening (112) has a diameter in a range from 0.01 mm to 0.5 mm, and
wherein at least a portion of the impact device (130) is formed integrally with the nozzle head (111).

2. Medical instrument (100) according to claim 1, wherein the impact body (131) has a parabolic shape, triangular shape, trapezoidal shape, or pyramidal shape, preferably a stepped pyramidal shape, in longitudinal section.

3. Medical instrument (100) according to one of claims 1 or 2, wherein the impact device (130) has a holding device (132) on which the impact body (131) is arranged, wherein the holding device (132) is preferably L-shaped or U-shaped, wherein the impact body (131) is arranged at a first end (133) of the holding device (132), wherein the second end (134) of the holding device (132) is attached to the nozzle head (111) and/or the shaft (120).

4. Medical instrument (100) according to claim 3, wherein a charging device (150) for electrostatically charging the fluid therapeutic substance is arranged on the holding device (132).

5. Medical instrument (100) according to claim 4, wherein the charging device (150) is arranged on a side opposite to the impact body (131) on the holding device (132).

6. Medical instrument (100) according to claim 4 or 5, wherein the charging device (150) comprises a conductive foil (151).

7. Medical instrument (100) according to claim 6, wherein a region of the conductive foil (151) is exposed to form a charging region (152) for electrostatically charging the fluid therapeutic substance.

8. Medical instrument (100) according to one of the preceding claims, wherein the nozzle head (111) has a plug connection (113) at an end opposite the nozzle opening (112), and wherein the tube (140) is plugged directly onto the plug connection (113).

9. Medical instrument (100) according to claim 8, wherein the medical instrument (100) has a clamping sleeve (160) arranged on the tube (140) in such a way that the clamping sleeve (160) engages circumferentially with its inner surface (161) against an outer surface (115) of the plug connection (113) on an end (141) of the tube (140) that is plugged onto the plug connection (113) with its inner surface (161) against an outer surface (115) of the plug connection (113).

10. Medical instrument (100) according to claim 9, wherein the shaft (120) has a preferably circumferential stop area (122) against which the clamping sleeve (160) abuts.

11. Medical instrument (100) according to one of the preceding claims, wherein the medical instrument (100) has a particle filter (170) which is preferably arranged in the nozzle (110).

12. Medical instrument (100) according to one of the preceding claims, wherein a bore (123) in the shaft (120) is formed to an outer diameter (142) of the tube (140) as a clearance fit such that the tube (140) is movable, in particular rotatable, within the shaft (120).

13. Medical instrument (100) according to one of the preceding claims, wherein the medical instrument (100) further comprises an adjustable outer stop (180) for positioning in a trocar (200) and a handle (190) arranged on the shaft (120).

14. Medical instrument (100) according to any of the preceding claims, wherein the nozzle (110) has a machine-readable identifier (116) containing information about the type of nozzle (110).

15. System comprising a trocar (200) and a medical instrument (100) according to claim 13, wherein the medical instrument (100) extends through the trocar (200).

## Revendications

1. Instrument médical (100) pour introduire une substance thérapeutique fluide dans une cavité (510) d'un corps (500), l'instrument médical (100) présentant:
une buse (110) avec une tête de buse (111) pour pulvériser la substance thérapeutique fluide à introduire, sans addition de gaz;
une tige (120) au moins sensiblement allongée et au moins sensiblement rigide, à une extrémité (121) de laquelle est disposée la tête de buse (111); et
un tuyau flexible (140) qui est disposé à l'intérieur de la tige allongée (120) et est relié fluidiquement à la tête de buse (111),
**caractérisé en ce que**
la buse (110) comprend un dispositif d'impact (130) qui présente un corps d'impact (131) qui est disposé devant une ouverture de buse (112) de la tête de buse (111) de manière au moins sensiblement coaxiale à l'ouverture de buse (112),
l'ouverture de buse (112) ayant un diamètre dans une plage de 0,01 mm à 0,5 mm, et
au moins une partie du dispositif d'impact (130) étant formée en une seule pièce avec la tête de buse (111).

2. Instrument médical (100) selon la revendication 1, le corps d'impact (131) présentant, en coupe longitudinale, une forme parabolique, triangulaire, trapézoïdale ou une forme pyramidale, de préférence une forme pyramidale étagée.

3. Instrument médical (100) selon l'une des revendications 1 ou 2, le dispositif d'impact (130) présentant un dispositif de retenue (132) sur lequel est disposé le corps d'impact (131), le dispositif de retenue (132) étant de préférence réalisé cintré en forme de L ou en forme de U, le corps d'impact (131) étant disposé à une première extrémité (133) du dispositif de retenue (132), la deuxième extrémité (134) du dispositif de retenue (132) étant montée sur la tête de buse (111) et/ou sur la tige (120).

4. Instrument médical (100) selon la revendication 3, un dispositif de charge (150) étant disposé sur le dispositif de retenue (132) pour charger électrostatiquement la substance thérapeutique fluide.

5. Instrument médical (100) selon la revendication 4, le dispositif de charge (150) étant disposé sur le dispositif de retenue (132) sur un côté opposé au corps d'impact (131).

6. Instrument médical (100) selon la revendication 4 ou 5, le dispositif de charge (150) présentant une feuille conductrice (151).

7. Instrument médical (100) selon la revendication 6, une zone de la feuille conductrice (151) étant exposée afin de former une zone de charge (152) afin de charger électrostatiquement la substance thérapeutique fluide.

8. Instrument médical (100) selon l'une quelconque des revendications précédentes, la tête de buse (111) comportant une fiche de raccordement (113) à une extrémité opposée à l'ouverture de buse (112), et le tuyau flexible (140) étant directement enfilé sur la fiche de raccordement (113).

9. Instrument médical (100) selon la revendication 8, l'instrument médical (100) présentant une douille de serrage (160) qui est disposée sur le tuyau flexible (140) de telle sorte que la douille de serrage (160) presse une extrémité (141) du tuyau flexible (140) enfilée sur la fiche de raccordement (113) de manière périphérique par sa surface d'enveloppe intérieure (161) contre une surface d'enveloppe extérieure (115) de la fiche de raccordement (113).

10. Instrument médical (100) selon la revendication 9, la tige (120) présentant une zone de butée (122), de préférence périphérique, contre laquelle vient buter la douille de serrage (160).

11. Instrument médical (100) selon l'une quelconque des revendications précédentes, l'instrument médical (100) comprenant un filtre à particules (170), qui est de préférence disposé dans la buse (110).

12. Instrument médical (100) selon l'une quelconque des revendications précédentes, un alésage (123) dans la tige (120) par rapport à un diamètre extérieur (142) du tuyau flexible (140) étant conçu comme un ajustement avec jeu de telle sorte que le tuyau flexible (140) est disposé de manière mobile, en particulier de manière rotative, à l'intérieur de la tige (120).

13. Instrument médical (100) selon l'une quelconque des revendications précédentes, l'instrument médical (100) comprenant en outre une butée extérieure réglable (180) pour le positionnement dans un trocart (200) et une poignée (190), qui sont disposées sur la tige (120).

14. Instrument médical (100) selon l'une quelconque des revendications précédentes, la buse (110) présentant un identificateur (116) lisible par machine, dans lequel sont contenues des informations relatives au type de la buse (110).

15. Système comprenant un trocart (200) et un instrument médical (100) selon la revendication 13, l'instrument médical (100) s'étendant à travers le trocart (200).
